(19) Europäisches Patentamt
European Patent Office
Office européen des brevets

(11) Veröffentlichungsnummer: **0 408 887 A2**

(12) **EUROPÄISCHE PATENTANMELDUNG**

(21) Anmeldenummer: **90111015.5**

(51) Int. Cl.[5]: **C07H 15/04**, C07C 235/08

(22) Anmeldetag: **11.06.90**

(30) Priorität: **14.07.89 DE 3923365**

(43) Veröffentlichungstag der Anmeldung:
**23.01.91 Patentblatt 91/04**

(84) Benannte Vertragsstaaten:
**AT BE CH DE DK ES FR GB GR IT LI NL SE**

(71) Anmelder: **SÜDZUCKER AKTIENGESELLSCHAFT MANNHEIM/OCHSENFURT**
**Maximilianstrasse 10**
**D-6800 Mannheim 1(DE)**

(72) Erfinder: **Hahn, Karola**
**Schwerdstrasse 20**
**D-6522 Osthofen(DE)**
Erfinder: **Schneider, Bernd, Dr. rer. nat.**
**Mertesheimer Strasse 25 a**
**D-6719 Ebertsheim(DE)**

(74) Vertreter: **Körber, Wolfhart, Dr. et al**
**Patentanwälte Mitscherlich & Partner**
**Steinsdorfstrasse 10 10**
**D-8000 München 22(DE)**

(54) **Oligosaccharidalditole mit Amidbindung sowie Verfahren zu ihrer Herstellung.**

(57) Die Erfindung betrifft Oligosaccharidalditole mit Amidbindung entsprechend der Formel

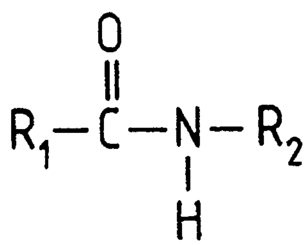

(I)

worin bedeuten:
$R_1$ = Alditolteil eines Mono-, Di- oder Oligosaccharidalditols, in dem der Alditolteil 2 bis 6 Kohlenstoffatome aufweist,
$R_2$ = Alditolteil eines Mono-, Di- oder Oligosaccharidaltols, in dem der Alditolteil 3 bis 7 Kohlenstoffatome aufweist und mit dem endständigen oder einem anderen Kohlenstoffatom verknüpft ist.

Ihre Herstellung erfolgt durch N-Acylierung von aus Sacchariden abgeleiteten Aminopolyolen mit Glyconsäuren in Anwesenheit von Methanol oder in aprotisch polaren Lösungsmitteln.

EP 0 408 887 A2

## OLIGOSACCHARIDALDITOLE MIT AMIDBINDUNG SOWIE VERFAHREN ZU IHRER HERSTELLUNG

Die vorliegende Erfindung betrifft Oligosaccharidalditole mit Amidbindung, im folgenden Amidooligosaccharide genannt, entsprechend der in Anspruch 1 sowie in den Ansprüchen 2 bis 4 genannten Formeln I, II, III, IV sowie die Herstellung derselben durch an sich bekannte N-Acylierung von aus Sacchariden abgeleiteten Aminopolyolen mit Glyconsäuren, wobei die Säurekomponente an der Carboxylfunktion aktiviert ist. Amidooligosaccharide, gebildet durch Reaktion von diesen Aminopolyolen mit den Glyconsäuren, sind eine in der Literatur noch nicht beschriebene Verbindungsklasse. Amidooligosaccharide entsprechen der allgemeinen Formel

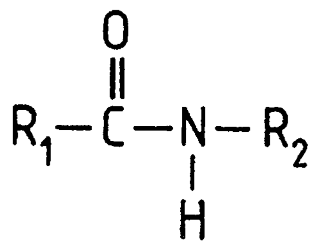

(I)

worin bedeuten:

$R_1$ = Alditolteil eines Mono-, Di- oder Oligosaccharidalditols, in dem der Alditolteil 2 bis 6 Kohlenstoffatome aufweist,

$R_2$ = Alditolteil eines Mono-, Di- oder Oligosaccharidalditols, in dem der Alditolteil 3 bis 7 Kohlenstoffatome aufweist und mit dem endständigen oder einem anderen Kohlenstoffatom verknüpft ist.

Die Konstitution der erhaltenen und beanspruchten Verbindungen entsprechend den Formeln

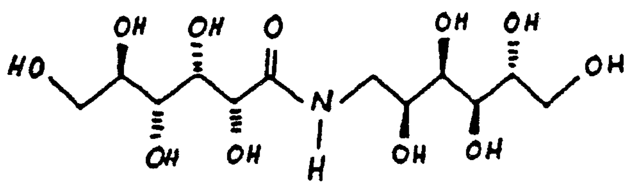

(II)

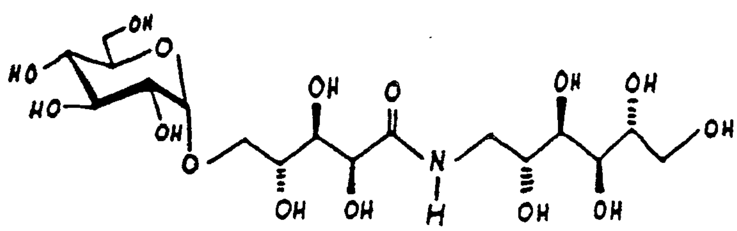

(III)

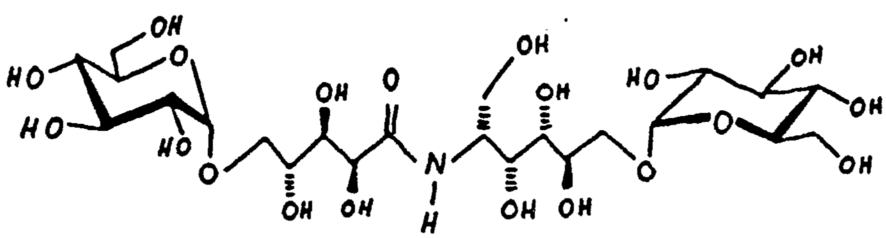

(IV)

ist durch $^{13}C$-NMR-spektroskopische Daten abgesichert.

Aminopolyole, wie z.B. Glucamin, Mannamin, Maltamin oder Isomaltamin, werden durch reduktive Aminierung von reduzierenden Mono-, Di- oder Oligosacchariden wie beispielsweise Glucose, Mannose, Fructose, Sorbose, Arabinose, Maltose, Isomaltose, Maltulose, Isomaltulose, Trehalulose, Laktose, Cellobiose und höhere Oligosaccharide sowie reduzierende Desoxyzucker, wie beispielsweise Rhamnose und C-verzweigte Kohlenhydrate in Ammoniak oder Hydrazin mit Wasserstoff unter Nickel-Katalyse erhalten (vgl. DE 35 38 451 und DE 36 25 931).

Glyconsäuren sind über verschiedene oxidative Verfahren aus reduzierenden Kohlenhydraten leicht zugänglich, z.B. Gluconsäure, Arabonsäure oder Glucopyranosylarabon säure (vgl. Übersicht: J. Stanek,

M. Cerný,

, J. Kocourek und J. Pacák, "The Monosaccharides", Academic Press, New York, 1963, S. 138 ff., und dort zit. Literatur, sowie Meth. Carbohydr. Chem. II, 11-26 (1963); Spengler, -O., Pfannenstiel, A.: Z. Zuckerind. 85 (1935), 546; D.R.P. 618 164, 62o 248 (1934); DE-OS 32 48 404, EP O 114 954).

Zur Reaktion zum Amidooligosaccharid muß die Säurekomponente an der Carboxylfunktion aktiviert werden; dies kann z. B. als Ester, Säurehalogenid oder Lacton erfolgen.

Die Verknüpfungsreaktion erfolgt in alkoholischer Lösung (vorzugsweise Methanol) oder in aprotisch polaren Lösungsmitteln (vorzugsweise Dimethylformamid) bei Temperaturen zwischen 20° und 120° C, vorzugsweise bei 60° C.

Die Additionsprodukte, entsprechend der Formel I, fallen während der Reaktion oder beim Abkühlen des Lösungsmittels in fester Form aus und können durch Umkristallisation weiter gereinigt werden.

Im Falle, daß kein abtrennbares Produkt in fester Form ausfällt, kann nach Verdampfung des Lösungsmittels der Rückstand durch chromatographische Verfahren aufgetrennt werden. Hierbei hat sich besonders die Gelchromatographie mit Wasser als Eluens bewährt, da die beanspruchten Verbindungen entsprechend ihrer Molekulargröße als erste Fraktion eluieren, während nicht umgesetzte Ausgangsprodukte längere Retentionszeiten aufweisen und getrennt wiedergewonnen werden können.

Die Konstitution der erhaltenen Amidooligosaccharide ist durch $^{13}C$-NMR-spektroskopische Daten abgesichert.

Beispiel 1: Umsetzung von Glucamin mit Gluconsäurelacton

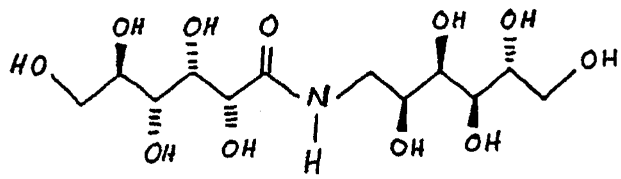

Je 5,3 g (30 mmol) Glucamin und Gluconsäurelacton werden zusammen in 100 ml wasserfreiem Methanol unter Erwärmung gelöst. Der Reaktionsansatz wird ca. 8 h unter Rückflußkühlung erhitzt, wobei nach einigen Stunden kristallines Disaccharid ausfällt. Der Niederschlag wird abgesaugt, mit kaltem Methanol gewaschen und im Vakuum bei 40° C getrocknet. Die Ausbeute betrug 8 g (76 %). Es wurde ein Schmelzpunkt von 145-148° C und eine optische Drehung von $[\alpha]_D^{20}$ = +12 (c=1; $H_2O$) gefunden.

Beispiel 2: Umsetzung von Mannamin mit Glucopyranosylarabonsäurelacton

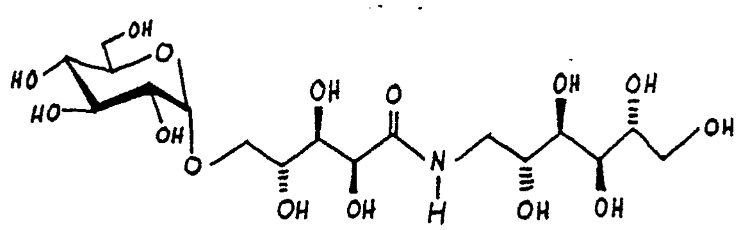

0,5 g (3,0 mmol) Mannamin und 1 g (3,0 mmol) Glucopyranosylarabonsäurelacton werden zusammen in 15 ml wasserfreiem Methanol gelöst und unter Rückflußkühlung erhitzt. Nach 8 h Reaktionszeit werden die farblosen Kristalle des Trisaccharides abgesaugt, aus heißem Methanol umkristallisiert und im Vakuum bei

40 °C getrocknet. Die Ausbeute betrug 0,7 g (47 %). Es wurde ein Schmelzpunkt von 191-195 °C und eine optische Drehung von $[\alpha]_D^{20} = +22$ ($c = 0,5$; $H_2O$) gefunden.

Beispiel 3: Umsetzung von Isomaltamin (Mannit-Isomeres) mit Glucopyranosylarabonsäurelacton

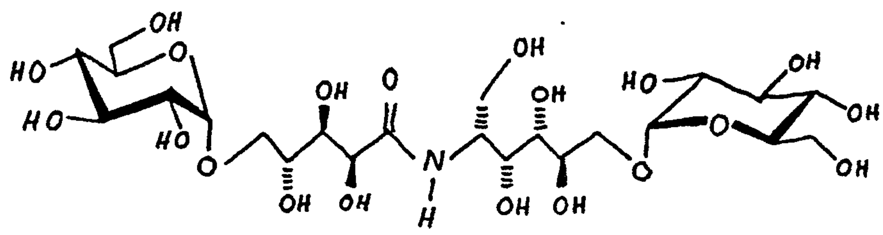

...

15 g (40 mmol) Isomaltamin (Mannit-Isomeres) und 12,4 g (40 mmol) Glucopyranosylarabonsäurelacton werden zusammen in 100 ml Dimethylformamid gelöst und 5 h bei 100 °C gerührt. Der Reaktionsansatz wird im Vakuum bis zur Trockne eingeengt.

Zur Abtrennung des Tetrasaccharides wird eine Gelchromatographie durchgeführt. Man erhält nach dem Einengen und Trocknen einen nichtkristallinen Schaum. Die Ausbeute betrug 13,2 g (46 %). Es wurde ein Schmelzpunkt von 70-72 °C und eine optische Drehung von $[\alpha]_D^{20} = +96$ ($c = 1$; $H_2O$) gefunden.

## Ansprüche

1. Oligosaccharidalditole mit Amidbindung entsprechend der Formel

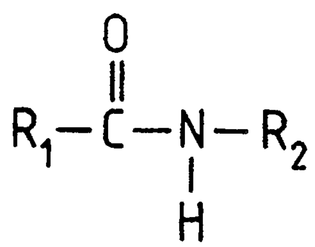

(I)

worin bedeuten:

$R_1$ = Alditolteil eines Mono-, Di- oder Oligosaccharidalditols, in dem der Alditolteil 2 bis 6 Kohlenstoffatome aufweist,

$R_2$ = Alditolteil eines Mono-, Di- oder Oligosaccharidalditols, in dem der Alditolteil 3 bis 7 Kohlenstoffatome aufweist und mit dem endständigen oder einem anderen Kohlenstoffatom verknüpft ist.

2. Oligosaccharidalditol mit Amidbindung gemäß Anspruch 1 entsprechend der Formel

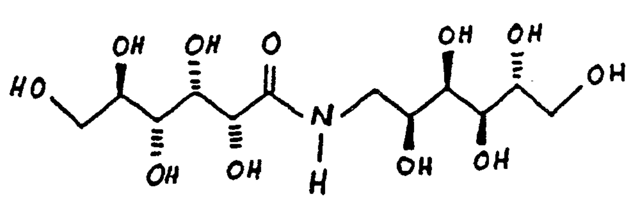

(II)

3. Oligosaccharidalditol mit Amidbindung gemäß Anspruch 1 entsprechend der Formel

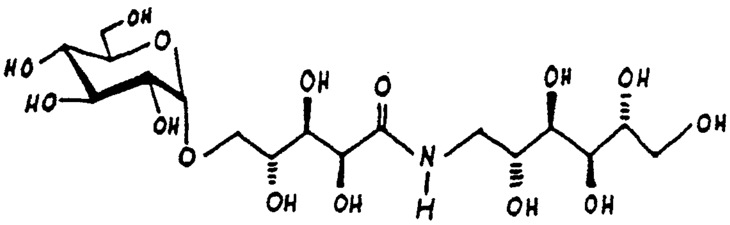

(III)

4. Oligosaccharidalditol mit Amidbindung gemäß Anspruch 1 entsprechend der Formel

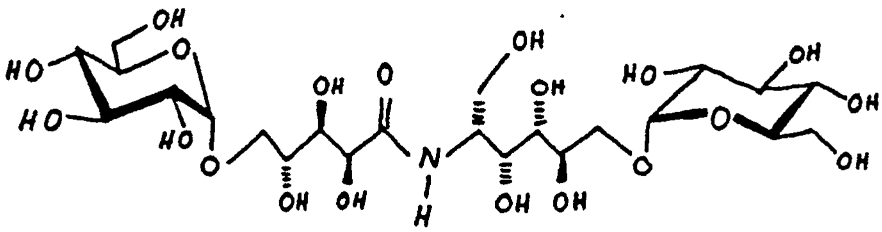

(IV)

5. Verfahren zur Herstellung von Oligosaccharidalditolen gemäß einem der Ansprüche 1 bis 4, gekennzeichnet durch
N-Acylierung von Aminopolyolen, die in an sich bekannter Weise über eine reduktive Aminierung von reduzierenden Mono-, Di- oder Oligosacchariden gewonnen wurden, in einem Lösungsmittel wie Methanol oder in aprotisch polaren Lösungsmitteln, wie Dimethylformamid, mit einem Glyconsäureanhydrid, -ester, -halogenid oder -lacton,
ggfs. in Gegenwart einer basischen Substanz, bei Temperaturen zwischen 20 °C und 120 °C, vorzugsweise bei 60 °C, mit anschließender Isolierung und Reinigung der so erhaltenen Verbindungen entweder durch Fällung, Kristallisation, Extraktion oder durch chromatographische Verfahren.